# EUROPEAN PATENT APPLICATION

(11) **EP 3 772 333 A1**
(43) Date of publication of application: **10.02.2021**
(21) Application number: 19191023.1
(22) Date of filing: 09.08.2019
(51) Int. Cl.: A61B 8/00, A61B 8/08

(54) **MUSCLE IMAGING SYSTEM**

(71) Applicant: Koninklijke Philips N.V., 5656 AG Eindhoven (NL)
(72) Inventor: WANG, Jim, 5656 AE Eindhoven (NL); JIN, Sheng, 5656 AE Eindhoven (NL); XU, Jingping, 5656 AE Eindhoven (NL); MENG, Yishuang, 5656 AE Eindhoven (NL)
(74) Representative: de Haan, Poul Erik

(57) **Abstract**

A muscle imaging system that uses an ultrasound probe to image one or more muscles of a subject. A processing unit controls a driving mechanism to set a position of the ultrasound probe with respect to the subject. A position of the ultrasound probe, during an imaging process, is at least partly based upon a shape of the subject detected by a shape sensor.

## Description

### FIELD OF THE INVENTION

The present invention relates to the field of obtaining information about a subject's muscles, and in particular, obtaining and processing images of a subject's muscles.

### BACKGROUND OF THE INVENTION

It is common for post-stroke subjects, or subjects living with multiple sclerosis (MS), to have movement difficulties. This is due to a flaccidity or spasticity of the subject's muscles preventing or hindering voluntary movement of the subject. It is important to track or assess the progress/state of the subject, e.g. post-stroke, in order to tailor treatment of the subject. In particular, there is an ongoing desire to accurately assess the condition of a subject's muscles, which reflects potential movement difficulties the subject may face.

Typically, the assessment of a subject's muscle is performed by a skilled clinician, such as a physician or therapist, manually observing or measuring a subject's muscles to fill out an assessment tool, which may also be called a scale, survey instrument or questionnaire table. The assessment tool provides an interpretation score to assess the subject's functionalities. Examples of suitable assessment tools include the "Brunnstrom Approach", the "Fugl-Meyer Assessment of Sensorimotor Function" (FMA) scale or the "Modified Ashworth scale" (MAS).

However, manual assessment methods are time and resource consuming, and can be inaccurate, e.g. due to inaccurate measurements by the clinician. A proposed solution is to use ultrasound or other medical images of a subject's muscles to (automatically) perform measurements of the subject's muscles. However, this still requires a time and resource intensive imaging process to be performed by the clinician, which may not be performed accurately.

There is therefore an ongoing desire to reduce the time and resources required to obtain, and improve an accuracy of obtaining, measurements of a subject's muscles.

### SUMMARY OF THE INVENTION

The invention is defined by the claims.

According to examples in accordance with an aspect of the invention, there is provided a muscle imaging system for obtaining ultrasound data about one or more muscles of a subject. The muscle imaging system comprises: an ultrasound probe adapted to perform an imaging process, the imaging process comprising imaging one or more muscles of the subject to thereby acquire ultrasound data of the one or more muscles; a driving mechanism adapted to adjust a position of the ultrasound probe during the imaging process; a shape sensor adapted to sense a shape of the subject; and a processing unit adapted to control the driving mechanism, during the imaging process, based on the sensed shape of the subject.

Thus, there is a muscle imaging system in which control over the position of an ultrasound probe is performed automatically based on determined shape of the subject (e.g. an arm or a leg). As the shape of the subject is defined by their muscles, by controlling the ultrasound probe during the imaging process based on their shape, the ultrasound probe can better track and image the muscle(s) of the subject. This improves the accuracy and ease of imaging the muscle(s) of the subject.

The shape of a subject is the external bounds or outline of the subject. This may be represented by a 3D contour image of the subject (e.g. as constructed by a shape sensor of an embodiment). If the shape of the subject is known, then the location of the external bounds of the subject is also known, so that the ultrasound probe can be positioned on, at or relative to the external bounds of the subject. In particular, the shape sensor may be adapted to determine a shape of the subject relative to (e.g. from the viewpoint of) the ultrasound probe. The ultrasound probe may, therefore, be automatically maintained in contact with the external bounds of the subject throughout an imaging process.

The present invention provides an automated way for a muscle imaging system to image one or more muscles of the subject.

The shape sensor may comprise, for example, a structured light sensor, a stereo camera and/or one or more proximity sensor.

The shape sensor may be adapted to sense a shape of the muscles of the subject. The shape of the subject is defined by the shape of the muscles of the subject. Thus, by sensing a shape of the subject, a shape and location of the muscles of the subject can be identified and the ultrasound probe can be positioned to follow the shape of the muscles.

The processing unit may be adapted to control the driving mechanism so that the ultrasound probe is maintained at a surface of the skin of the subject.

The location of the skin of the subject can be calculated or determined from shape sensor, so that the shape of the subject is detected by the shape sensor, Thus, the processing unit is capable of keeping the ultrasound probe at a surface of the skin of the subject (e.g. in contact with the subject). This means that the ultrasound imaging can take place with improved accuracy.

In some embodiments, the shape sensor is adapted to track a distance between the shape sensor and the surface of the subject, to thereby determine a shape of the surface of the subject. This effectively enables a depth map of the subject to be built up by the shape sensor, to thereby determine a shape of the subject. This provides an adaptable and low-resource (e.g. cheap) method of determining a shape of the subject. The depth map allows the processing unit to control the driving mechanism so that the ultrasound probe is maintained at a certain distance (e.g. in contact with) the subject - as the relative position of the ultrasound probe with respect to the calculated depth map can be determined.

In some embodiments, the driving mechanism is adapted to adjust the position of the ultrasound probe in at least a first and second orthogonal axis; the shape sensor is adapted to track a distance between the shape sensor and the surface of the subject along the second orthogonal axis, wherein an offset with respect to the second orthogonal axis, between the shape sensor and the ultrasound probe is known or can be readily calculated; and the processing unit is adapted to control the driving mechanism, during the imaging process, to iteratively adjust a position of the ultrasound probe, wherein: the magnitude of the iterative adjustment to the position of the ultrasound probe is predefined in the first orthogonal axis; and the iterative adjustment to the position of the ultrasound probe in the second orthogonal axis is controlled to maintain the ultrasound probe in contact with the surface of the subject.

In some embodiments, the processing unit is adapted to iteratively adjust a position of the ultrasound probe using the driving mechanism. The imaging process performed by the ultrasound probe may comprise acquiring an ultrasound image of the subject between each iterative adjustment of the ultrasound probe.

Thus, the ultrasound probe may obtain a series or sequence of ultrasound images at different locations of the subject. This series or sequence of ultrasound images forms the ultrasound data of the one or more muscles, and can be later used to reconstruct a 3D ultrasound image by a post-processing unit/module.

In some embodiments, the driving mechanism is adapted to adjust the position of the ultrasound probe in at least two orthogonal axes; and the processing unit is adapted so that, during the imaging process, the magnitude of the iterative adjustment to the position of the ultrasound probe is predefined in at least one axis and variable, based on the sensed shape of the subject, in at least one other axis.

Thus, the movement of ultrasound probe may be predefined in a first orthogonal axis, for example, X-axis, with the movement of the ultrasound probe in a second and/or third orthogonal axis/axes (Y-axis or Z-axis) being based upon the sensed shape (e.g. to account for the movement in the first axis).

In other words, the ultrasound probe may be moved along a particular direction of travel with respect to the subject (e.g. along a first orthogonal axis), with a vertical and/or side-to-side movement of the ultrasound probe being controlled based upon the detected shape of the subject.

This may, for example, enable the ultrasound probe to be maintained in (good) contact with the skin of the subject as the ultrasound probe is moved down or up the subject, along the X-axis.

The processing unit may be adapted to receive a user input defining each predefined magnitude of the iterative adjustment in the at least one axis. Thus, a user may be able to define how the ultrasound probe moves in a certain direction of travel, e.g. the interval between different ultrasound images being taken.

In some embodiments, the driving mechanism is adapted to adjust the position of the ultrasound probe in at least a first and second orthogonal axis; and the shape sensor is connected to the driving mechanism so that an adjustment to the position of the ultrasound probe along the first orthogonal axis induces a corresponding movement in the shape sensor along the first orthogonal axis.

In some embodiments, the shape sensor may be connected to the driving mechanism so that a movement of the ultrasound probe in the first orthogonal axis induces an equivalent movement of the subject sensor in that axis. This effectively allows the shape sensor to track or scout ahead of the ultrasound probe of a movement in that axis, to determine the shape of the subject before an ultrasound probe reaches that location. This allows an appropriate location for the ultrasound probe along the second and/or third orthogonal axes to be calculated with respect to the tracked shape of the subject.

The shape sensor may be adapted to determine a shape of the subject as the shape sensor is moved, during the imaging process, by the driving mechanism.

Thus, the shape of the subject may be gradually learnt or built up as the driving mechanism moves the ultrasound probe. In particular, the shape sensor may move ahead of the ultrasound probe, i.e. to investigate potential future locations for the ultrasound probe, to establish a shape of the subject at the potential future locations for the ultrasound probe.

As the shape sensor is moved, it can build up a profile image of the subject to determine appropriate locations or co-ordinates for the ultrasound probe. By way of example, information from the shape sensor may be capable of identifying locations for the ultrasound probe in which the ultrasound probe is in contact with a surface of the subject's skin.

In other embodiments, the shape of the subject is learnt before performing the imaging process, e.g. by performing a scanning process on the subject. This increases the time required to image the subject (as the scanning process needs to be performed), but may increase an accuracy of determining the shape of the subject and for pre-emptively determining suitable locations for positioning the ultrasound probe.

Optionally, the driving mechanism is adapted to adjust the position of the ultrasound probe in a first and second orthogonal axis; and the processing unit is adapted to control the driving mechanism, during the imaging process, by performing an iterative process comprising: obtaining a next position along the first orthogonal axis for the ultrasound probe; determining a next position along the second orthogonal axis for the ultrasound probe based on the determined shape for the subject at the next position along the first orthogonal axis; and controlling the driving mechanism to place the ultrasound probe at a single location lying at the next position along the first orthogonal axis and the next position along the second orthogonal axis.

Thus, the processing unit may determine a position for the probe along at least one axis perpendicular to the direction of movement of the ultrasound probe.

Preferably, the driving mechanism is further adapted to adjust the position of the ultrasound probe in a third axis, orthogonal to the first and second orthogonal axes; the iterative process performed by the processing unit comprises determining a next position along the third orthogonal axis for the ultrasound probe based on the determined shape for the subject at the next position along the first orthogonal axis; and the step of controlling the driving mechanism, in the iterative process performed by the processing unit, comprises controlling the driving mechanism to place the ultrasound probe at a single location lying at the next position along the first orthogonal axis, the next position along the second orthogonal axis and the next position along the third orthogonal axis.

In some embodiments, the step of obtaining the next position for the ultrasound probe with respect to the first orthogonal axis comprises receiving a user input indicating a next position with respect to the first orthogonal axis. The user input may, for example, define a predetermined distance of movement for the ultrasound probe along the first orthogonal axis.

In at least one embodiment, the muscle imaging system further comprises a frame that mounts at least the ultrasound probe, the driving mechanism and the shape sensor.

The muscle imaging system may further comprise a user interface adapted to receive a user input, e.g. for use as any previously described user input. The user interface may comprise, for example, a keyboard, screen, mouse and so on.

There is also proposed a muscle assessment system comprising any previously described muscle imaging system; and a post-processing unit adapted to: receive the ultrasound data of the one or more muscles; and process the ultrasound data to determine one or more parameters of the one or more muscles. A parameter of the one or more muscles may include a dimension or measurement of one or more features of the muscle(s).

The one or more parameters of the muscle may include any one or more of: an area of each one or more muscles; a volume of each one or muscles; a perimeter of each one or more muscles; a diameter of each one or more muscles; and a radius of each one or more muscles. These provide examples of suitable dimensions or measurements of one or more features of the muscles.

The post-processing unit may process the ultrasound data using one or more image segmentation technique to determine the one or more parameters.

According to examples in accordance with another aspect of the invention, there is provided a computer-implemented method of obtaining information about one or more muscles of a subject. The method comprises: sensing a shape of the subject; performing an imaging process comprising using an ultrasound probe to image one or more muscles of the subject to thereby acquire ultrasound data of the one or more muscles; and, during the imaging process, using a driving mechanism to adjust a position of the ultrasound probe based on the sensed shape of the subject. The step of sensing a shape of the subject may comprise using a shape sensor that moves as the position of the ultrasound probe moves to sense the shape of the subject.

According to examples in accordance with yet another aspect of the invention, there is provided computer program comprising code means for implementing any described method when said program is run on a processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a generic ultrasound system for understanding a context of the present invention;
Figure 2 illustrates a muscle assessment system comprising a muscle imaging system according to an embodiment;
Figure 3 illustrates a profile image generated by a shape sensor of an embodiment; and
Figure 4 illustrates a method of using a muscle assessment system according to an embodiment.

### DETAILED DESCRIPTION OF THE EMBODIMENTS

The invention will be described with reference to the Figures.

It should be understood that the detailed description and specific examples, while indicating exemplary embodiments of the system, systems and methods, are intended for purposes of illustration only and are not intended to limit the scope of the invention. These and other features, aspects, and advantages of the system, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a muscle imaging system that uses an ultrasound probe to image one or more muscles of a subject. A processing unit controls a driving mechanism to set a position of the ultrasound probe with respect to the subject. A position of the ultrasound probe, during an imaging process, is at least partly based upon a shape of the subject detected by a shape sensor.

Embodiments are at least partly based on the realization that muscles of a subject define the shape of the subject. Thus, it is appreciated that the shape of the subject may be detected using a shape sensor in order to improve a placement of the ultrasound probe for imaging the subject's muscles, i.e. with greater accuracy and ease.

Illustrative embodiments may, for example, be employed in a muscle assessment apparatus for easing one or more parameters or measurements of a subject's muscles. This can be used to improve an ease and accuracy of obtaining measurements of a subject's muscles, e.g. for calculating an interpretation score of a medical assessment tool.

Throughout this document, reference to an X-axis, Z-axis and Y-axis may be replaced by the terms "first orthogonal axis", "second orthogonal axis" and "third orthogonal axis" respectively. The X, Z and Y axes are orthogonal (i.e. perpendicular) to one another.

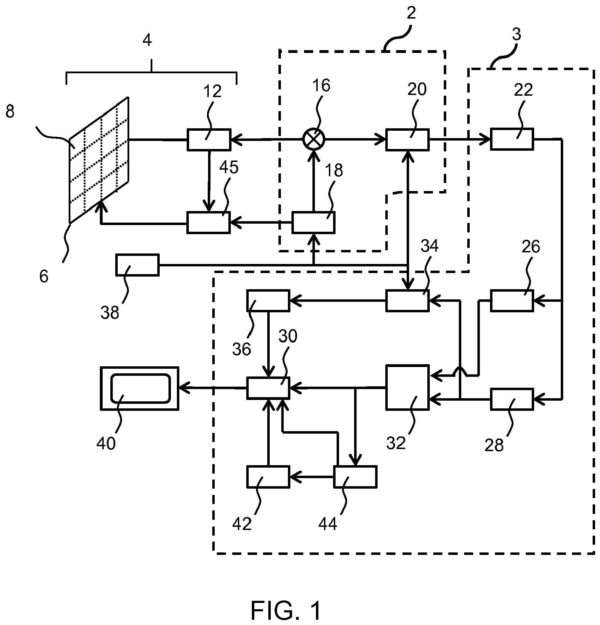

The general operation of an exemplary ultrasound system will first be described, with reference to Figure 1. The system comprises control circuit 2, processing circuitry 3 and a transducer probe 4.

The transducer probe 4 comprises a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a (optional) microbeamformer 12 that controls reception of signals by the transducers. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

The control circuitry 2 includes a transmit/receive (T/R) switch 16, to which the (optional) microbeamformer 12 maybe coupled. The T/R switch 16 switches the array between transmission and reception modes, and protects the main beamformer 20 from high energy transmit signals in the case where a microbeamformer is not used and the transducer array is operated directly using the main system beamformer.

The transmission of ultrasound beams from the transducer array 6 is directed by a transducer controller 18 coupled to the (optional) microbeamformer 12 by the T/R switch 16 and a main transmission beamformer 20, which can receive input from the user's operation of a user interface or control panel 38. The transducer controller 18 may also include transmission circuitry arranged to drive the transducer elements of the array 6 (either directly or via a microbeamformer) during the transmission mode.

In a typical line-by-line imaging sequence, the beamforming system within the probe may operate as follows.

During transmission, the beamformer 12, 20 (which may be the microbeamformer 12 or the main beamformer 20 depending upon the implementation) activates the transducer array 6, or a sub-aperture of the transducer array. The sub-aperture may be a one-dimensional line of transducers or a two-dimensional patch of transducers within the larger array 6. In transmit mode, the focusing and steering of the ultrasound beam generated by the array, or a sub-aperture of the array, are controlled as later described.

Upon receiving the backscattered echo signals from the subject, the received signals undergo receive beamforming (later described), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during a receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

The transducer controller 18 controls the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to focusing, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

In transmit mode, only one focus may be defined unless the ultrasound image is divided into multiple focal zones (each of which may have a different transmit focus).

In addition, upon receiving the echo signals from within the subject, it is possible to perform the inverse of the above-described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22 of the processing circuitry 3. The signal processor 22 can process the received echo signals in various ways, such as: band-pass filtering; decimation; I and Q component separation; and harmonic signal separation, which separates linear and nonlinear signals to identify nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass-band sliding from a higher to a lower frequency band as echo signals are received from increasing depths, thereby rejecting noise at higher frequencies from greater depths that is typically devoid of anatomical information.

The beamformers for transmission and for reception are implemented in different hardware and can have different functions. The receiver beamformer is designed to take characteristics of the transmission beamformer into account. For simplicity, in Figure 1, only the receiver beamformers 12, 20 are shown. In the complete system, a transmission chain with a transmission micro beamformer, and a main transmission beamformer may be present.

The micro beamformer 12 provides an initial combination of signals in order to decrease the number of analog signal paths. This is typically performed in the analog domain. The main beamformer 20 performs the final beamforming, and is typically after digitization.

The transmission and reception channels use the same transducer array 6, which has a fixed frequency band. However, the bandwidth that the transmission pulses occupy can vary depending on the transmission beamforming used. The reception channel can either capture the whole transducer bandwidth or, using bandpass processing, extract only the bandwidth containing the desired information, e.g. harmonics of the main harmonic.

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue, muscles and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. The scan converter may act to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

A user interface 38 may be also coupled to the transmit controller 18 to control the generation of ultrasound signals from the transducer array 6 and hence the images produced by the transducer array and the overall ultrasound system. The controller 18 may also take account of the mode of operation (given by the user) and the corresponding required transmitter configuration and band-pass configuration in the receiver analog to digital converter. The controller 18 can be a state machine with fixed states.

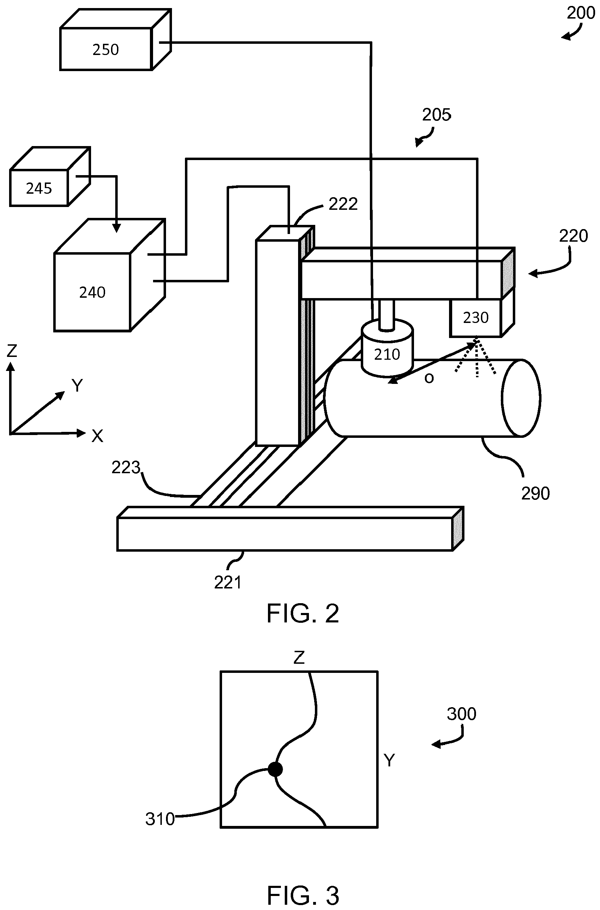

Figure 2 illustrates a muscle assessment system 200, comprising a muscle imaging system 205, and a post-processing unit 250, according to an embodiment of the invention.

The muscle imaging system 205 comprises an ultrasound probe 210, a driving mechanism 220, a shape sensor 230 and a processing unit 240.

The ultrasound probe 210 is adapted to perform an imaging process on a subject 290, e.g. such as any imaging process previously described, to capture ultrasound data of one or more muscles of the subject. The ultrasound data may be processed to generate an ultrasound image, e.g. using control circuitry and processing circuitry previously described. The control and/or processing circuitry for the ultrasound probe may be implemented in the ultrasound probe, the processing unit 240 or a separate module (not shown).

By way of example only, the imaging process may comprise iteratively capturing 2D ultrasound images of the subject 290. Thus, the imaging process may comprise generating a series or sequence of ultrasound images of one or more muscles of the subject, said series/sequence acting as the ultrasound data.

In another example, the imaging process may comprise capturing a 2-dimensional video of the subject, i.e. a 2D video of one or more muscles of the subject.

The driving mechanism 220 is adapted to adjust a position of the ultrasound probe 210 during the imaging process. Put another way, the driving mechanism 220 is able to move the ultrasound probe 210 relative to the imaged subject 290 at least whilst the ultrasound probe 210 is imaging the subject 290.

The illustrated driving mechanism 220 is adapted to adjust a position of the ultrasound probe with respect to three orthogonal axes X, Z, Y, i.e. within a Euclidean space. In particular, the driving mechanism may define the co-ordinates of the ultrasound probe with respect to the three orthogonal axes X, Z, Y.

In an embodiment, this is performed by the driving mechanism 220 moving the ultrasound probe 230 along one or more tracks 221, 222, 223 of the driving mechanism 220, e.g. a respective track positioned along each orthogonal axis. A first track 221 may define the X-axis (or first orthogonal axis) position of the ultrasound probe, a second track 222 may define the Z-axis (or second orthogonal axis) position of the ultrasound probe and a third track 223 may define the Y-axis (or third orthogonal axis) position of the ultrasound probe.

In embodiments, the orthogonal axes X, Y, Z need not lie along the direction of the tracks. This is because the tracks allow the movement of the ultrasound probe within a 3D space, so that the position (i.e. orientation or direction) of the orthogonal axes can be arbitrary within this 3D space.

Other methods of moving an ultrasound probe within three orthogonal axes (i.e. within a 3D space) will be apparent to the skilled person, e.g. using a mechanical arm. In some embodiments, the driving mechanism only moves the ultrasound probe with respect to two orthogonal axes, i.e. within a single plane.

The subject 290 to be imaged preferably lies substantially along the X-axis. The subject may, for example, be an arm or a leg (i.e. a limb). In some embodiments, the position of the subject 290 may define the location of X-axis. For example, the X-axis may be defined as an axis along which the subject (e.g. arm or leg) lies, with the Y-axis and Z-axis being defined relative to this.

The shape sensor 230 is adapted to sense a shape of the subject 290, i.e. the relative location of one or more external bounds of the subject. In particular examples, the shape sensor may construct a 3D contour image of the subject, in particular the muscles of the subject. The determined shape may be a shape of the subject for potential future locations/positions (in one or more axes) of the ultrasound probe. Examples of suitable shape sensors include a proximity sensor, an array of proximity sensors, a structured light sensor, a time-of-flight sensor and so on.

The shape sensor 230 may be capable of determining a distance between the shape sensor and the body in order to determine a depth map representing the shape of the subject. In other embodiments, the shape sensor may determine a shape of the subject independent of distance from the shape sensor or ultrasound sound, e.g. a shape relative to a starting point of shape determination.

Determining a shape of the subject means that the relative location of the external bounds of the subject is known. This enables the ultrasound probe to be automatically positioned relative to the known external bounds of the subject during the ultrasound process, e.g. to maintain contact with the external bounds (skin) of the subject.

In the illustrated embodiment, the shape sensor is mounted on the driving mechanism 220 so that movement of the ultrasound probe 210 induces an equivalent movement in the shape sensor.

In other embodiments, the shape sensor 230 is mounted on the driving mechanism 220 so that a movement of the ultrasound probe 210 with respect to at least one orthogonal axis (but not necessarily all three) induces an equivalent movement of the shape sensor 230 in the same at least one axis.

In particular, the shape sensor 230 may be mounted so that at least a movement of the ultrasound probe 210 along the X-axis induces an equivalent movement of the shape sensor 230 along the X-axis. This means that, as the ultrasound probe is moved along the X-axis (e.g. during the imaging process), the shape sensor is moved along the X-axis to enable it to determine a shape of the subject.

Preferably, the shape sensor 230 is mounted so that a movement (i.e. change of position) of the ultrasound probe 210 in any of the three orthogonal axes induces an identical movement (or change of position) of the shape sensor 230.

The shape sensor 230 is mounted so that a distance or offset o, with respect to at least one axis X, between the shape sensor 230 and the ultrasound probe 210 is known or can be readily calculated. This effectively enables the shape of the subject relative to (e.g. from the viewpoint of) the ultrasound probe to enable appropriate positioning of the ultrasound prove with respect to the shape of the subject.

For example, if the shape sensor is not positioned to move with a Z-axis movement of the ultrasound probe, the relative position of the shape sensor on the Z-axis may be known and used to calculate the Z-axis distance between the ultrasound probe and the sensor as the ultrasound probe moves up and down the Z-axis.

In a first example, the shape sensor 230 is a structured light sensor comprising a camera and a laser source adapted to use a triangular measurement principle to obtain the Z distance (i.e. a distance along the Z-axis) between the shape sensor 230 and the surface of the subject. This allows a depth map to be constructed.

The structured light sensor may, for example, project a predetermined pattern onto the surface of the subject, and monitor a change or deformity of the projected pattern to identify a distance and/or shape of the subject below the shape sensor. The projected predetermined pattern may be a one-dimensional pattern (e.g. substantially linear) or a two-dimensional pattern.

In a second example, the shape sensor 230 is a stereo camera adapted to construct a 3D contour image of the muscles. The usage of a stereo camera for determining a shape or outline of a structure is well known in the art. The stereo camera can determine how different areas of the subject are positioned relative to one another.

In a third example, the shape sensor 230 is a proximity sensor adapted to determine a distance (along a Z-axis) of the subject from the shape sensor 230. The shape sensor may comprise a 1-dimensional or 2-dimensional proximity sensor (or array of proximity sensors), so that a depth map of the area of the subject below the shape sensor 230 can be constructed. The depth map can be iteratively constructed (e.g. stitched together) as the shape sensor is moved over the surface of the subject, e.g. via movement of the ultrasound sensor.

Examples of proximity sensors include a photoelectric (e.g. light or infrared) or other optical sensor, a capacitive proximity sensor or an ultrasonic sensor.

By way of example, the shape sensor may comprise a 1-dimensional proximity sensor (or 1-dimensional array of proximity sensors) arranged perpendicularly to the X-axis and in a line parallel to the Y-axis.

The 1-dimensional proximity sensor may capture a Z-axis distance between the subject and the shape sensor along a line parallel to the Y-axis. Thus, as the ultrasound probe is moved along the X-axis, a Z-axis distance is calculated for a plurality of Y-axis positions along each X-axis position. In particular, for a particular X-axis position, the 1-dimensional proximity sensor will determine a Z-axis distance (between the subject and the shape sensor) for each of a plurality of Y-axis positions.

The shape sensor may be capable of obtaining at least a 1-dimensional vector of Z-axis distances. In particular, at a particular X-axis position, the shape sensor may be capable of determining a Z-axis distance for each of a plurality of Y-axis positions, to thereby determine a shape of the subject at the X-axis position. Thus, if the shape sensor is moved along the X-axis, an overall shape of the subject is built up.

In a preferred embodiment, the shape sensor is capable of obtaining a 2-dimensional array of Z-axis distances. In particular, the shape sensor may (simultaneously) obtain at least a Z-axis distance for a plurality of Y-axis positions and corresponding X-is positions, i.e. at a 2-dimensioanl array of Y and X-axis positions. In this way, a shape of the subject at plurality of X-axis positions is obtained.

The X-axis positions (and/or Y-axis positions) may be spaced a predetermined distance apart.

Thus, the shape sensor is able to track or otherwise determine a (external) shape of the subject as the ultrasound probe 210 is moved along the X-axis (e.g. during the imaging process). In particular, more information about the shape of the subject is obtained as the shape sensor moves about the subject, which movement is induced by a movement of the ultrasound probe, e.g. during the imaging process.

By sensing the shape of the subject, the shape sensor is simultaneously able to determine a shape of the muscles of the subject. It will be apparent that the external shape of a subject is dictated by the location, size and shape of the muscles of the subject. By tracking the shape of the subject in this manner, the shape of the muscles of the subject can be tracked at the same time.

The processing unit 240 is adapted to control the driving mechanism, to thereby control a position of at least the ultrasound probe 210, during the imaging process. In particular, the processing unit 240 is adapted to a position of the ultrasound probe (with respect to one or more of the orthogonal axes X, Y, Z) based on the sensed shape of the subject.

The processing unit may be able to learn (from the shape sensor 230) the shape of the subject, and in particular, a shape of the subject surrounding a next desired location (e.g. with respect to a particular axis) for the ultrasound probe to be positioned for imaging the subject. The processing unit may then appropriately position the ultrasound probe at the next location based on the shape of the subject.

By way of example, the processing unit may be adapted to keep the ultrasound probe in (good) contact with a skin surface of the subject.

Where the shape sensor constructs a depth map, the Z-axis distance between the shape sensor and the surface of the (skin of the) subject is known for a plurality of Y-axis positions associated with a desired X-axis position. As an offset between the shape sensor and ultrasound probe is also known (or can be calculated), when the ultrasound probe is moved to a certain X and Y-axis position, the Z-axis position of the ultrasound probe can be controlled so that the ultrasound probe contacts the skin of the subject.

In some embodiments, the Z-axis position may be selected to be slightly below the detected surface of the skin (e.g. 0.5-10mm, depending upon the imaged location), so that the ultrasound probe pushes against the skin. This ensures that good contact with the skin is made and maintained.

By way of another example, the processing unit may be adapted to keep the ultrasound probe in contact with a line or contour of a muscle, which may be made clear from the determined shape of the subject. As will be later explained, the location of the muscle may be associated with the shortest Z-axis distance, so that iteratively positioning the ultrasound probe at the Y-axis position having the shortest Z-axis position (for the next desired X-axis position) may cause the ultrasound probe to track or follow the line of the muscle.

The precise operation of the processing unit 240, i.e. how the ultrasound probe 210 is controlled, will vary in different embodiments. This variation may also define a required operation of the shape sensor 230, and the level of detail required for the shape of the subject.

In a relatively simple first scenario, the processing unit 240 is adapted to continuously or iteratively move the ultrasound probe 210 along a single direction of the X-axis during an imaging process, with the Y-axis position being held constant and the Z-axis position being based upon the sensed shape of the subject 290. In particular, the Z-axis position may be controlled to keep the ultrasound probe 210 in contact with the subject 290.

In other words, the X-axis co-ordinate of the ultrasound probe may be independent of the sensed shape of the subject (e.g. based on a user input), the Y-axis co-ordinate may be fixed throughout the imaging process, and the Z-axis co-ordinate may be derived from the sensed shape of the subject.

In this first scenario, the shape sensor 230 need only be capable of determining a distance (i.e. along the Z-axis) between the shape sensor 230 (or ultrasound probe) and the subject 290 (at a position along the same Y-axis in which the ultrasound probe lies). In particular, the shape sensor 230 is positioned to track ahead of the movement of the ultrasound probe along the X-axis, to thereby track the shape (location of the external bounds) of the subject in advance of the movement of the ultrasound probe. Thus, the shape sensor (or processing unit) can build up a 1-dimensional depth profile of the subject along the X-axis.

When the ultrasound probe 210 moves to an X-axis position for which the shape sensor 230 has measured a corresponding distance (along the Z-axis), the processing unit may be adapted to move the Z-axis position of the ultrasound probe 210 so that it remains in contact with or at a surface of the skin of the subject (e.g. rather than attempting to lift off the skin of the subject or press into the skin of the subject). In another example, the processing unit may be adapted to move the Z-axis position of the ultrasound probe 210 to that it is positioned a certain distance (e.g. 0.5-5mm) below the surface of the subject, to ensure good contact with the subject is maintained.

The offset in the Z-axis between the shape sensor 230 and ultrasound probe 210 is fixed (or can be otherwise calculated). In this way, the appropriate Z-axis position for the ultrasound probe can be set based on the determined distance associated with the appropriate X-axis position.

In this first scenario, the driving mechanism 220 only needs to be capable of moving the ultrasound probe in two orthogonal axes (the X and Z axes).

Thus, in the first scenario, the processing unit is adapted to control the driving mechanism, during the imaging process, by performing an iterative process comprising: obtaining a next position along the first orthogonal axis for the ultrasound probe; determining a next position along the second orthogonal axis for the ultrasound probe based on the determined shape for the subject at the next position along the first orthogonal axis; and controlling the driving mechanism to place the ultrasound probe at a single location lying at the next position along the first orthogonal axis and the next position along the second orthogonal axis.

In a more complex second scenario, the processing unit is adapted to continuously or iteratively move the ultrasound probe along a single direction of the X-axis during an imaging process, with the Y-axis and Z-axis positions being based upon the sensed shape of the subject 290.

In other words, the X-axis co-ordinate of the ultrasound probe may be independent of the sensed shape of the subject (e.g. provided by a user input) and the Y-axis and Z-axis co-ordinates may be derived from the sensed shape of the subject.

In this second scenario, the shape sensor 230 should be at least capable of determining a distance (i.e. along the Z-axis) between the shape sensor 230 and the subject 290 for a plurality of different Y-axis positions (e.g. for each possible Y-axis position).

In a first example, when the ultrasound probe 210 moves to an X-axis position for which the shape sensor 230 has measured a corresponding distance (along the Z-axis) for a plurality of Y-axis positions, the processing unit 240 may be adapted to move the ultrasound probe 210 so that is lies at a Y-axis position associated with the shortest/smallest Z-axis distance between the shape sensor the subject, and so that the ultrasound probe lies at an appropriate Z-axis position for it to remain at the surface of the skin of the subject.

In a second example, when the ultrasound probe 210 moves to an X-axis position for which the shape sensor 230 has measured a corresponding distance (along the Z-axis) for a plurality of Y-axis positions, the processing unit 240 may be adapted to move the ultrasound probe 210 so that is lies at a Y-axis position within a predetermined range of Y-axis positions (e.g. from a previous Y-axis position of the ultrasound probe) associated with the shortest Z-axis distance between the shape sensor the subject, and so that the ultrasound probe lies at an appropriate Z-axis position for it to remain at the surface of the skin of the subject. By limiting the Y-axis position to lie within a predetermined range of Y-axis positions, sudden jumps of the ultrasound probe can be avoided, so that the line of a muscle can continue to be tracked.

By way of example, medical objects positioned on/in a subject's arm or leg (e.g. a plaster or cannula) may provide a false reading for the Z-axis distance, which can be avoided by limiting the subsequent Y-axis positions to lie within a predetermined range of Y-axis positions from a previous Y-axis position.

The predetermined range of Y-axis positions may be a range equal to the previous Y-axis position ± a predetermined distance. The predetermined distance may be approximately equal to (e.g. ± 1% or ±5%) or less than a distance between a new X-axis position and a previous X-axis position, as it is unlikely that a muscle will deviate by more than this value within the Y-axis, e.g. if the subject is aligned with the X-axis.

The shape sensor 230 may determine a shape of the subject during the imaging process or prior to the imaging process.

Figure 3 illustrates a profile image 300 for a particular X-axis position, e.g. generated by the shape sensor, in which a Z-axis distance has been determined for each of a plurality of Y-axis positions. The processing unit may be arranged to effectively place the ultrasound probe at a point 310 associated with the smallest Z-axis distance.

In this way, the position of the ultrasound probe can track the position of smallest Z-axis distance. This effectively results in the position of the ultrasound probe tracking the position of the muscle of the subject, as muscle is typically located below the point having the smallest Z-axis distance (i.e. the greatest bulge or outward contour of the subject).

In this way, the ultrasound probe effectively tracks the muscle to thereby automatically image the entirety of the muscle. This improves the imaging of the muscle, and thereby any subsequent determination of parameters of the subject's muscle.

In particular, the adaptive adjustment to the Y-axis and Z-axis position of the ultrasound probe means that, as the X-axis position of the ultrasound probe is changed, the ultrasound probe remains at the center of a muscle having a variable shape. This means that a more complete ultrasound scan of the muscle can be automatically performed, e.g. without the need for intervention from a clinician. This significantly reduces a burden on the clinician for performing ultrasound imaging of a muscle of the subject.

Thus, in the second scenario, the processing unit is adapted to control the driving mechanism, during the imaging process, by performing an iterative process comprising: obtaining a next position along the first orthogonal axis for the ultrasound probe; determining a next position along the second orthogonal axis for the ultrasound probe based on the determined shape for the subject at the next position along the first orthogonal axis; determining a next position along the third orthogonal axis for the ultrasound probe based on the determined shape for the subject at the next position along the first orthogonal axis; and controlling the driving mechanism to place the ultrasound probe at a single location lying at the next position along the first orthogonal axis, the next position along the second orthogonal axis and the next position along the third orthogonal axis.

In the foregoing embodiments, the processing unit may be adapted to iteratively move the ultrasound probe along the X-axis, e.g. at intervals between a start and end location which may be defined by the user. The imaging process performed by the ultrasound probe may comprise capturing an ultrasound image after each iterative movement.

The distance of the iterative movement (i.e. the sampling interval) controlled by the processing unit 240 may be predefined, e.g. by a user. In particular, the processing unit may receive a user input indicating a predefined X-axis distance. The processing unit may then iteratively move the position of the ultrasound probe (along the X-axis) this predefined X-axis distance, with the Z-axis position (and optionally Y-axis position) being based on the data form the shape sensor.

In another example, the processing unit 240 may be adapted to continuously move the ultrasound probe along the X-axis, e.g. between a start and end location which may be defined by the user. The imaging process performed by the ultrasound probe may comprise iteratively capturing a (2D) ultrasound image during the continuous movement or capturing a 2D ultrasound video during the continuous movement.

For improved imaging accuracy, it is preferred to capture an ultrasound image of the subject when the ultrasound probe is stationary relative to the subject, e.g. after an iterative movement rather than during a continuous movement. There may therefore be a pause between each iterative movement to enable the ultrasound probe to capture a (2D) ultrasound image.

The processing unit 240 may be adapted to communicate with a user interface 245 (which may form part of the muscle imaging system 200) to determine the sampling interval, the start location and/or the end location. The user interface may comprise an input mechanism for a user, e.g. a keyboard, screen, mouse and so on.

The user interface 245 may be adapted to receive a user input indicating a distance for the ultrasound probe to move along the X-axis (thereby defining the end location) and a sampling interval (i.e. the distance between iterative movements of the ultrasound probe). In another example, the user input may indicate a start X-axis position, an end X-axis position and the sampling interval.

The user interface 245 may pass the information contained in the user input to the processing unit 240, which can subsequently control the ultrasound probe based on this information and the determined shape of the subject as previously described.

Referring back to Figure 2, the muscle assessment system 200 may also comprise a post-processing unit 250 for processing the ultrasound data (e.g. ultrasound image(s)) generated by the ultrasound probe 210 of the muscle imaging system 205. Thus, the post-processing unit 250 may receive ultrasound data from the ultrasound probe 210 for processing.

The post-processing unit 250 is adapted to process the ultrasound data to as to generate or determine one or more parameters of the imaged area of the subject. In particular, the post-processing unit 250 processes the ultrasound data to determine one or more parameters of the one or more muscles. Exemplary parameters include dimensions of the one or more muscles, such as a width, height, area, volume, circumference, radius and diameter (amongst other dimensions).

In one embodiment, the ultrasound data comprises a series or sequence of 2D ultrasound images. The post-processing unit may be adapted to reconstruct a 3D ultrasound image based on the series or sequence of ultrasound images. This may be performed by stacking the 2D ultrasound images and extrapolating between the stacked 2D ultrasound images, as is well known in the art.

In another embodiment, the ultrasound data obtained from the ultrasound probe may itself comprise a 3D ultrasound image of the subject, e.g. constructed by circuitry within the ultrasound probe.

After obtaining a 3D ultrasound image of the subject, the post-processing unit 250 may be adapted to perform muscle detection using image segmentation techniques and/or artificial intelligence systems (such as neural networks). Muscle detection from a 3D ultrasound image is known in the art.

The post-processing unit may also be adapted to further process the detected muscles in the 3D ultrasound image to calculate one or more parameters or dimensions or the muscle. Methods of determining parameters based on detected muscles within a 3D ultrasound image are also well known in the art.

An example of determining a muscle stiffness using ultrasound imaging is disclosed in Wu, Chueh-Hung, et al. "Evaluation of post-stroke spastic muscle stiffness using shear wave ultrasound elastography." Ultrasound in medicine & biology 43.6 (2017): 1105-1111.

In another example, methods for determining a muscle corrected echogenicity and muscle thickness were used by Berenpas, Frank, et al. in "Bilateral changes in muscle architecture of physically active people with chronic stroke: a quantitative muscle ultrasound study." Clinical Neurophysiology 128.1 (2017): 115-122.

In a further example, a method for detecting acromion-greater tuberosity (AGT) distance using ultrasound is used in Idowu, Bukunmi M., et al. "Sonographic detection of inferior subluxation in post-stroke hemiplegic shoulders." Journal of ultrasonography 17.69 (2017): 106.

From at least the foregoing, it is evident that the concept of determining parameters based on detected muscles within ultrasound images are well known in the art.

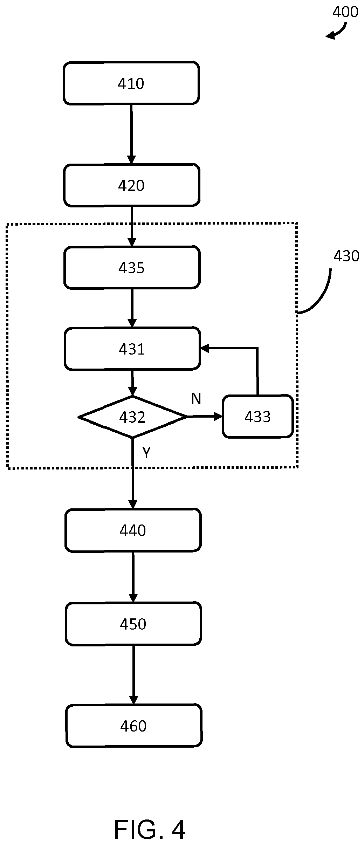

Figure 4 illustrates a method 400 or workflow of operating a muscle assessment system according to an embodiment of the invention.

The method 400 comprises a first step 410 of positioning the ultrasound probe at a start location or position with respect to the subject.

Initially, the muscle imaging system may not know the shape of the subject, e.g. the shape sensor may not be able to determine the shape of the subject until the imaging process starts. Thus, the first step 410 may be performed by a clinician physically placing the ultrasound probe at an appropriate start location (e.g. in contact with the subject's skin).

The method 400 may then further comprise a second step 420 of providing a user input indicating a desired end location and a desired interval for iterative movements of the ultrasound probe. The second step 420 may also be performed by the clinician.

It will be appreciated that there is therefore a corresponding step (as an alternative to step 420) of receiving the user input, which is performed by the muscle imaging system 200 (in particular, the user interface 245 of the muscle imaging system).

The method 400 further comprises an imaging process 430, following step 420. The imaging process here comprises a step 431 of capturing a 2D ultrasound image.

In step 432, it is determined whether the ultrasound probe has reached the desired end location. In response to the ultrasound probe reaching the desired end location, the imaging process may end, and the method 400 may then move, for example, to a step 440. Otherwise, a step 433 of moving the ultrasound probe based on the determined shape of the subject is performed, and the imaging process 430 reverts back to step 431.

In some embodiments, step 433 may be the first step performed in the imaging process 430 (i.e. the next step performed after step 420 has been completed). In other words, the first ultrasound image may be captured after the positon of the ultrasound probe has been moved based on the determined shape of the subject.

The shape of the subject is sensed in a step 435. Step 435 may be performed during the imaging process (as illustrated), or may be performed before the imaging process. In one example, for each iterative movement of the ultrasound probe, the shape of the subject at potential subsequent locations/positions of the ultrasound probe may be determined and used to pick or select an appropriate subsequent location of the ultrasound probe.

Thus, the shape of the subject may be built up during the imaging process or determined prior to the imaging process. When performing during the imaging process, step 435 may be performed before, during and/or after the ultrasound probe is moved.

After performing the imaging process, the ultrasound data (i.e. the sequence of series of ultrasound images) captured during the imaging process may be processed, e.g. by the post-processing unit, in steps 440-460.

Step 440 may comprise reconstructing a 3D ultrasound image based on the series or sequence of ultrasound images. This may be performed by stacking the 2D ultrasound images and extrapolating between the stacked 2D ultrasound images, as is well known in the art. Step 450 may comprise performing muscle detection by using image segmentation techniques and/or artificial intelligence systems (such as neural networks) on the reconstructing 3D ultrasound image. Step 460 may comprise further processing the detected muscles in the 3D ultrasound image to calculate one or more parameters or dimensions of the muscle.

In this way, parameters or dimensions of the muscle can be determined. Steps 440-460 are optional, and may be omitted in some embodiments.

It is not essential that the imaging process comprise capturing a series of ultrasound images. Rather, a single 3D ultrasound image may be captured or constructed during the movement of the probe, according to known embodiments. It is also not essential that a 3D ultrasound image be constructed; rather a series of 2D ultrasound images may be captured. The series of 2D ultrasound images may itself be processed to identify parameters of the muscle(s) of the subject.

The skilled person would be readily capable of developing a processing system for carrying out any herein described method. Thus, different steps of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system, which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A muscle imaging system (205) for obtaining ultrasound data of one or more muscles of a subject (290) comprising:
an ultrasound probe (210) adapted to perform an imaging process, the imaging process comprising imaging one or more muscles of the subject to thereby acquire ultrasound data of the one or more muscles;
a driving mechanism (220) adapted to adjust a position of the ultrasound probe during the imaging process;
a shape sensor (230) adapted to sense a shape (300) of the subject; and
a processing unit (240) adapted to control the driving mechanism, during the imaging process, based on the sensed shape of the subject.

2. The muscle imaging system (205) of claim 1, wherein the shape sensor (230) is adapted to sense a shape of the muscles of the subject (290).

3. The muscle imaging system (205) of any of claims 1 or 2, wherein the processing unit (240) is adapted to control the driving mechanism (220) so that the ultrasound probe (210) is maintained at a surface of the skin of the subject.

4. The muscle imaging system (205) of any of claims 1 to 3, wherein:
the driving mechanism (220) is adapted to adjust the position of the ultrasound probe in at least two orthogonal axes (X, Z); and
the processing unit (240) is adapted to control the driving mechanism, during the imaging process, to iteratively adjust a position of the ultrasound probe, wherein the magnitude of the iterative adjustment to the position of the ultrasound probe is predefined in at least one axis and variable, based on the sensed shape of the subject, in at least one other axis.

5. The muscle imaging system (205) of any of claims 1 to 4, wherein the shape sensor (230) is adapted to track a distance between the shape sensor and the surface of the subject (290), to thereby determine a shape of the surface of the subject.

6. The muscle imaging system (205) of claim 5 wherein:
the driving mechanism (220) is adapted to adjust the position of the ultrasound probe (210) in at least a first (X) and second (Z) orthogonal axis;
the shape sensor (230) is adapted to track a distance between the shape sensor and the surface of the subject (290) along the second orthogonal axis, wherein an offset (o) with respect to the second orthogonal axis, between the shape sensor and the ultrasound probe is known or can be readily calculated; and
the processing unit (240) is adapted to control the driving mechanism, during the imaging process, to iteratively adjust a position of the ultrasound probe, wherein:
the magnitude of the iterative adjustment to the position of the ultrasound probe is predefined in the first orthogonal axis; and
the iterative adjustment to the position of the ultrasound probe in the second orthogonal axis is controlled to maintain the ultrasound probe in contact with the surface of the subject.

7. The muscle imaging system (205) of any of claims 1 to 6, wherein:
the driving mechanism (220) is adapted to adjust the position of the ultrasound probe (210) in at least a first (X) and second (Z) orthogonal axis; and
the shape sensor (230) is connected to the driving mechanism so that an adjustment to the position of the ultrasound probe in the first orthogonal axis induces a corresponding movement in the shape sensor in the first orthogonal axis.

8. The muscle imaging system (205) of claim 7, wherein the shape sensor (230) is adapted to determine a shape of the subject (290) as the shape sensor is moved along the first orthogonal axis (X), during the imaging process, by the driving mechanism (220).

9. The muscle imaging system (205) of any of claims 1 to 8, wherein:
the driving mechanism (220) is adapted to adjust the position of the ultrasound probe (210) in a first (X) and second (Z) orthogonal axis; and
the processing unit (240) is adapted to control the driving mechanism, during the imaging process, by performing an iterative process comprising:
obtaining a next position along the first orthogonal axis for the ultrasound probe;
determining a next position along the second orthogonal axis for the ultrasound probe based on the determined shape for the subject (290) at the next position along the first orthogonal axis; and
controlling the driving mechanism to place the ultrasound probe at a single location lying at the next position along the first orthogonal axis and the next position along the second orthogonal axis.

10. The muscle imaging system (205) of claim 9, wherein:
the driving mechanism (220) is further adapted to adjust the position of the ultrasound probe (210) in a third orthogonal axis (Y), being an axis orthogonal to both the first (X) and second (Z) orthogonal axes;
the iterative process performed by the processing unit (240) comprises determining a next position along the third orthogonal axis for the ultrasound probe based on the determined shape for the subject (290) at the next position along the first orthogonal axis; and
the step of controlling the driving mechanism, in the iterative process performed by the processing unit, comprises controlling the driving mechanism to place the ultrasound probe at a single location lying at the next position along the first orthogonal axis, the next position along the second orthogonal axis and the next position along the third orthogonal axis.

11. The muscle imaging system (205) of any of claims 9 or 10, wherein the step of obtaining the next position for the ultrasound probe with respect to the first orthogonal axis comprises receiving a user input indicating a next position with respect to the first orthogonal axis.

12. A muscle assessment system (200) comprising:
the muscle imaging system (205) of any of claims 1 to 11; and
a post-processing unit (250) adapted to:
receive the ultrasound data of the one or more muscles; and
process the ultrasound data to determine one or more parameters of the one or more muscles.

13. The muscle assessment system (200) of claim 12, wherein the one or more parameters of the muscle include one or more of: an area of each one or more muscles; a volume of each one or muscles; a perimeter of each one or more muscles; a diameter of each one or more muscles; and a radius of each one or more muscles.

14. A computer-implemented method (400) of obtaining ultrasound data about one or more muscles of a subject (290), the method comprising:
sensing (435) a shape of the subject;
performing (430) an imaging process comprising using an ultrasound probe to image (431) one or more muscles of the subject to thereby acquire ultrasound data of the one or more muscles;
during the imaging process, using a driving mechanism to adjust (433) a position of the ultrasound probe based on the sensed shape of the subject.

15. A computer program comprising code means for implementing the method of claim 14 when said program is run on a processing system.
